# EUROPEAN PATENT APPLICATION

(11) **EP 4 276 497 A1**
(43) Date of publication of application: **15.11.2023**
(21) Application number: 22172305.9
(22) Date of filing: 09.05.2022
(51) Int. Cl.: G01T 7/00, A61B 6/00

(54) **DETECTION AND COLLECTION OF X-RAY DETECTOR ARTEFACT DATA BY THE ACQUISITION SYSTEM TO SUPPORT FIELD SERVICE AND DEVELOPMENT**

(71) Applicant: Koninklijke Philips N.V., 5656 AG Eindhoven (NL)
(72) Inventor: KOEPNICK, Johannes, Eindhoven (NL); VAN DER HEIJDEN, Hendrik, Eindhoven (NL)
(74) Representative: Philips Intellectual Property & Standards

(57) **Abstract**

The present invention relates to X-ray imaging. In order to improve artefacts management, there is provided an anomaly detection apparatus for identifying disturbance in an X-ray detector image caused by an external source. The anomaly detection apparatus comprises an input unit, a processing unit, and an output unit. The input unit is configured to receive an unexposed image acquired by an X-ray detector and metadata associated with the acquired unexposed image. The processing unit is configured to analyse the received unexposed image to determine whether there is an anomaly in the received unexposed image that is indicative of an image disturbance caused by an external source. In response to the detection of an anomaly that is indicative of an image disturbance caused by an external source, the processing unit is configured to generate a single image anomaly report comprising the received unexposed image in full or data-size-reduced form and the received metadata. The output unit is configured to provide the generated single image anomaly report.

## Description

### FIELD OF THE INVENTION

The present invention relates to X-ray imaging. In particular, the present invention relates to an anomaly detection apparatus and an anomaly detection method for identifying disturbance in an X-ray detector image caused by an external source, to an X-ray detector, to a system, to a computer program, and to a computer-readable medium.

### BACKGROUND OF THE INVENTION

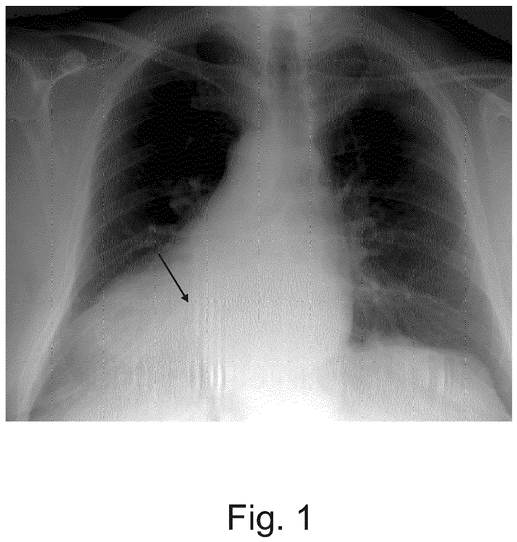

In conventional X-ray, an acquisition comprises acquiring an image pair including an X-ray frame with radiation and an offset frame without radiation. The subtraction of offset from the X-ray frame leads to an offset corrected X-ray image. However, some disturbances may vary overtime, such as electronic noise, electromagnetic interference, and mechanical vibrations. These disturbances cannot be corrected by the offset frame because the offset frame is acquired at a different time point, e.g., shortly before or after the X-ray. Therefore, visible artefacts may remain in the offset corrected X-ray image. For example, Fig. 1 shows an example of an X-ray clinical image with artefacts caused by mechanical vibration during acquisition.

### SUMMARY OF THE INVENTION

There may be a need to improve artefacts management.

The object of the present invention is solved by the subject-matter of the independent claims. Further embodiments and advantages of the invention are incorporated in the dependent claims. Furthermore, it shall be noted that all embodiments of the present invention concerning a computer-implemented method might be carried out with the order of the steps as described, nevertheless this has not to be the only and essential order of the steps of the method as presented herein. The computer implemented method disclosed herein can be carried out with another order of the disclosed steps without departing from the respective method embodiment, unless explicitly mentioned to the contrary hereinafter.

According to a first aspect of the present invention, there is provided an anomaly detection apparatus for identifying disturbance in an X-ray detector image caused by an external source. The anomaly detection apparatus comprises an input unit, a processing unit, and an output unit. The input unit is configured to receive an unexposed image acquired by an X-ray detector and metadata associated with the acquired unexposed image. The processing unit is configured to analyse the received unexposed image to determine whether there is an anomaly in the received unexposed image that is indicative of an image disturbance caused by an external source. In response to the detection of an anomaly that is indicative of an image disturbance caused by an external source, the processing unit is configured to generate a single image anomaly report comprising the received unexposed image in full or data-size-reduced form and the received metadata. The output unit is configured to provide the generated single image anomaly report.

The anomaly detection apparatus and method as described herein analyse the unexposed images acquired by the X-ray detector. The unexposed images are also referred to as dark images. A dark image is an image, sometimes called offset image or frame, acquired by the X-ray detector, which does not include signal (or include some residual signal from previous acquisitions, so-called Lag) from X-ray radiation. From the dark images, useful information can be obtained about the clinical environment and the effects on the detector images. This collected data may give useful insights about the present disturbance signals and support implementation or improvement of correction software for related artefacts. No data privacy rules must be considered because the dark images do not contain any patient related information. The mechanism may be either part of the X-ray detector or part of an external device like a computer connectable to the X-ray detector.

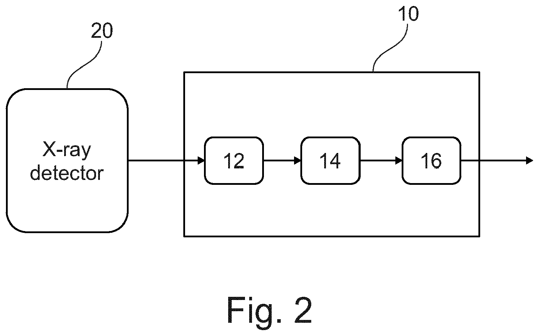

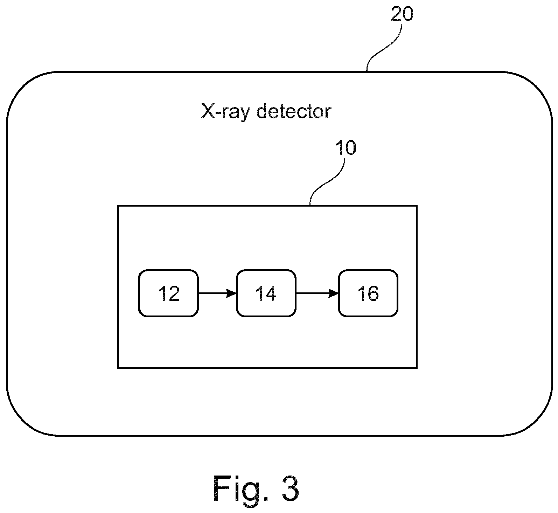

This will be explained in detail hereinafter and in particular with respect to the examples shown in Figs. 2 and 3.

According to an embodiment of the present invention, the processing unit is configured to determine the anomaly in the received unexposed image by comparing the received unexposed image with a set of reference unexposed images comprising one or more unexposed images that were previously acquired by the X-ray detector.

According to an embodiment of the present invention, the processing unit is further configured to analyse the detected anomaly and to provide analytical information about the detected anomaly to the single image anomaly report.

According to an embodiment of the present invention, the analytical information about the detected anomaly comprises one or more of:
- artefact type of the detected anomaly;
- signal frequencies derived from the artefact data;
- size and location information about the detected anomaly; and
- strength of the detected anomaly.

*As* the signature of the artefacts in the images depends on the signal form of the disturbance, *the artefact type of the detected anomaly may be a useful information to determine the type of external sources (e.g., mechanical vibration, electronic noises, or electromagnetic interference) that cause the disturbance.*

*The size and location information may be a useful information to make it easier to find the location of the external source.*

*The strength of the detected anomaly may indicate the strength* of the *influence* of the *external source.*

*The anomaly signal waveform derived from the image and its contained frequencies may be a useful information to identify the external source.*

According to an embodiment of the present invention, the processing unit is further configured to create a thumbnail image of the detected anomaly and to provide the created thumbnail image of the detected anomaly to the single image anomaly report.

According to an embodiment of the present invention, the processing unit is further configured to provide, via the output unit, instructions to prevent future anomalies.

*In some examples, the anomaly detection apparatus may store a lookup table storing a plurality of abnormalities, each being associated with one or more actions (which may be defined by one or more service technicians according to their experiences, or may be determined based on historic actions that were previously performed by one or more service technicians) to prevent future anomalies. The anomaly detection apparatus can then use the single image anomaly report to find the matching actions.*

*In some examples, the anomaly detection apparatus may send the single image anomaly report to a server, and the server returns instructions to the anomaly detection apparatus.*

According to an embodiment of the present invention, the processing unit is configured to provide the single image anomaly report to a data storage.

*The data storage may include a local storage, a network storage, and*/*or a cloud storage.*

According to an embodiment of the present invention, the metadata comprises one or more of: date and time of acquiring the unexposed image, customer identifier, detector identifier, the examination type, position of the X-ray detector when acquiring the unexposed image, orientation of the X-ray detector when acquiring the unexposed image, motion of the X-ray detector when acquiring the unexposed image, and temperature of the X-ray detector when acquiring the unexposed image.

According to a second aspect of the present invention, there is provided an X-ray detector. The X-ray detector comprises the anomaly detection apparatus according to the first aspect and any associated example.

In some examples, the X-ray detector may be a fixed detector.

In some examples, the X-ray detector may be a portable detector.

According to a third aspect of the present invention, there is provided a system. The system comprises the anomaly detection apparatus for identifying disturbance in an X-ray detector image caused by an external source according to the first aspect and any associated example, a data storage, and an anomaly analysis apparatus. The anomaly detection apparatus is configured to generate, for each unexposed image acquired by an X-ray detector having an anomaly that is indicative of an image disturbance caused by an external source, a respective single image anomaly report and to provide the said single image anomaly report to the data storage. The anomaly analysis apparatus is configured to analyse the single image anomaly reports stored in the data storage and to generate an anomaly summary report comprising analytical information about detected anomalies stored in the data storage.

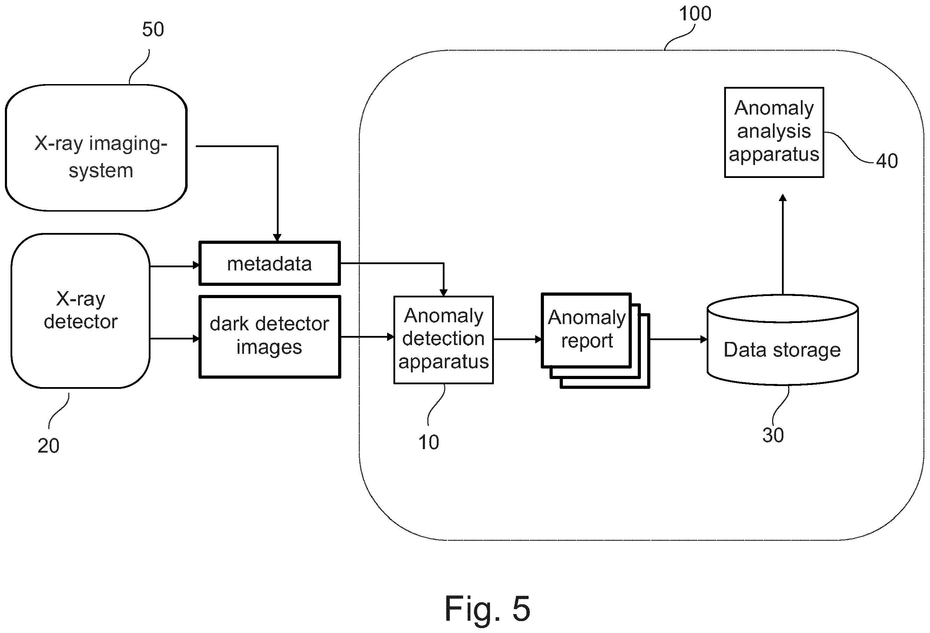

This will be explained in detail hereinafter and in particular with respect to the example shown in Fig. 5.

According to an embodiment of the present invention, the anomaly summary report comprises one or more of a list of detected anomalies stored in the data storage, and statistics comprising occurrence rate of anomalies.

According to an embodiment of the present invention, the action proposal comprises one or more of: suggest to investigate an artefact that is not corrected well, so that correction of affected images can be improved, inform customer that a recently added device disturbs the detector, inform customer that the artefact he is experiencing is the same as other customers get from a specific device, and inform customer that he has disturbances on a regular schedule.

According to a fourth aspect of the present invention, there is provided an anomaly detection method for identifying disturbance in an X-ray detector image caused by an external source, comprising:
- receiving an unexposed image acquired by an X-ray detector and metadata associated with the acquired unexposed image;
- analysing the received unexposed image to determine whether there is an anomaly in the acquired unexposed image that is indicative of an image disturbance caused by an external source;
- in response to the detection of an anomaly that is indicative of an image disturbance caused by an external source, generating a single image anomaly report comprising the received unexposed image in full or data-size-reduced form and the received metadata; and
- providing the generated single image anomaly report.

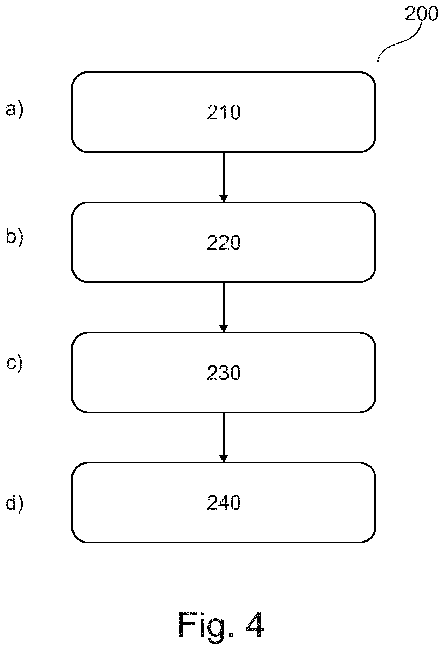

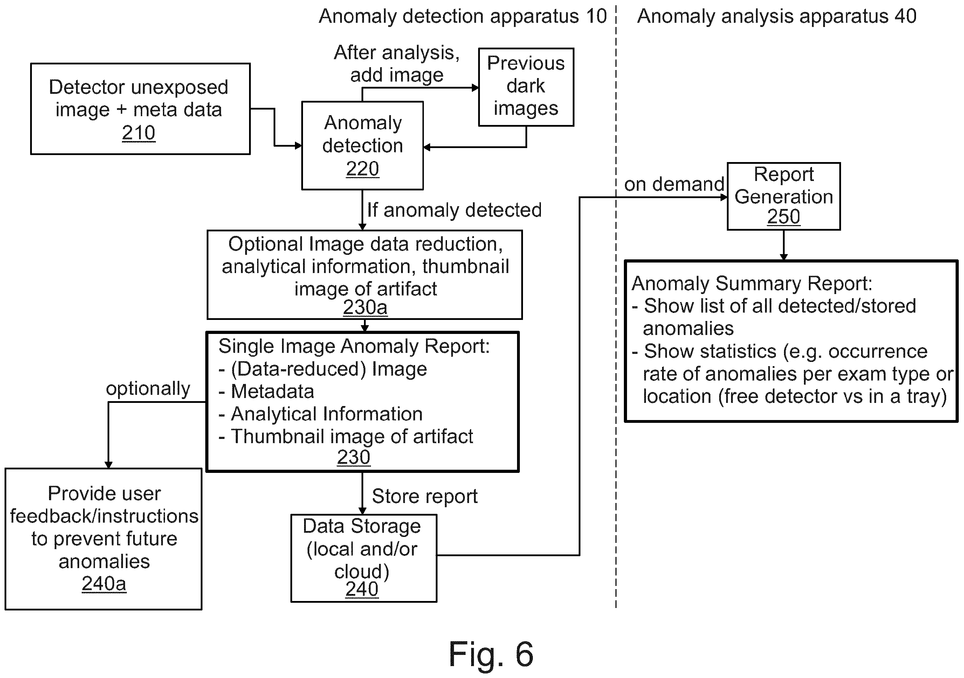

This will be explained in detail hereinafter and in particular with respect to the example shown in Figs. 4 and 6.

According to another aspect of the present invention, there is provided a computer program comprising instructions which, when the program is executed by a computer, cause the computer to carry out steps according to method of the fourth aspect.

According to a further aspect of the present invention, there is provided a computer-readable medium having stored thereon the computer program.

As used herein, the term "anomaly", also referred to as "image artefact", may refer to any abnormalities in images which can interfere with relevant image information. The image artefact may comprise detector typical artefacts and externally caused artefacts. Examples of the detector typical artefacts may include, but are not limited to, patterns due to inhomogeneities in pixel matrix and analogue electronics (e.g., photo diodes, amplifier chip) that may vary slowly with time and temperature, lag effects or memory effects caused by the radiation from previous X-ray acquisitions, noise such as analogue noise before and from the ADC. The externally caused artefacts may refer to the artefacts caused by electronic noise, electromagnetic interference, or mechanical shock/vibration. The externally caused artefacts may vary over time and cannot be corrected by the dark frame because the dark signal is acquired at different time point, which is shortly before or after the X-ray. Therefore, these externally caused artefacts may remain in the clinical image.

It should be appreciated that all combinations of the foregoing concepts and additional concepts discussed in greater detail below (provided such concepts are not mutually inconsistent) are contemplated as being part of the inventive subject matter disclosed herein. In particular, all combinations of claimed subject matter appearing at the end of this disclosure are contemplated as being part of the inventive subject matter disclosed herein.

These and other aspects of the invention will be apparent from and elucidated with reference to the embodiment(s) described hereinafter.

### BRIEF DESCRIPTION OF THE DRAWINGS

In the drawings, like reference characters generally refer to the same parts throughout the different views. Also, the drawings are not necessarily to scale, emphasis instead generally being placed upon illustrating the principles of the invention.
Fig. 1 shows an example of an X-ray clinical image with artefacts caused by mechanical vibrations during acquisition.
Fig. 2 illustrates a block diagram of an exemplary anomaly detection apparatus for identifying disturbance in an X-ray detector image caused by an external source.
Fig. 3 illustrates an alternate embodiment of an anomaly detection apparatus.
Fig. 4 illustrates a flow chart describing a computer-implemented method for identifying disturbance in an X-ray detector image caused by an external source.
Fig. 5 illustrates a block diagram of an exemplary system.
Fig. 6 illustrates a flow chart describing a further example of the computer-implemented method.

### DETAILED DESCRIPTION OF EMBODIMENTS

Fig. 2 illustrates a block diagram of an exemplary anomaly detection apparatus 10 for identifying disturbance in an X-ray detector image caused by an external source, in accordance with an embodiment. The anomaly detection apparatus 10 may receive an unexposed image acquired by an X-ray detector 20 and metadata associated with the acquired unexposed image. The X-ray detector 20 is used to convert X-rays into an image used for radiography. It may be a portable detector or a fixed detector.

The anomaly detection apparatus 10 may include an input unit 12, one or more processing units 14, and an output unit 16.

In general, the anomaly detection apparatus 10 may comprise various physical and/or logical components for communicating and manipulating information, which may be implemented as hardware components (e.g., computing devices, processors, logic devices), executable computer program instructions (e.g., firmware, software) to be executed by various hardware components, or any combination thereof, as desired for a given set of design parameters or performance constraints. Although Fig. 2 may show a limited number of components by way of example, it can be appreciated that a greater or a fewer number of components may be employed for a given implementation. Furthermore, the functions provided by one or more components of the anomaly detection apparatus 10 may be combined or separated.

In some implementations, the anomaly detection apparatus 10 may be embodied as, or in, a device or apparatus, such as a server, workstation, or mobile device. The anomaly detection apparatus 10 may comprise one or more microprocessors or computer processors, which execute appropriate software. The software may have been downloaded and/or stored in a corresponding memory, e.g., a volatile memory such as RAM or a non-volatile memory such as flash. The software may comprise instructions configuring the one or more processors to perform the functions as described herein.

It is noted that the anomaly detection apparatus 10 may be implemented with or without employing a processor, and also may be implemented as a combination of dedicated hardware to perform some functions and a processor (e.g., one or more programmed microprocessors and associated circuitry) to perform other functions. For example, the functional units of the anomaly detection apparatus 10, e.g., the input unit 12, the processing unit 14, and the output unit 16 may be implemented in the device or apparatus in the form of programmable logic, e.g., as a Field-Programmable Gate Array (FPGA). In general, each functional unit of the anomaly detection apparatus may be implemented in the form of a circuit.

In some examples, the anomaly detection apparatus 10 may be implemented by a computing platform such as a mobile platform, personal computer (PC) platform, and/or consumer electronics (CE) platform supporting various networking, communications, and/or multimedia capabilities. Such capabilities may be supported by various networks, such as a Wide Area Network (WAN), Local Area Network (LAN), Metropolitan Area Network (MAN), wireless WAN (WWAN), wireless LAN (WLAN), wireless MAN (WMAN), wireless personal area network (WPAN), Worldwide Interoperability for Microwave Access (WiMAX) network, broadband wireless access (BWA) network, the Internet, and/or any other wired or wireless network in accordance with the described embodiments.

Returning to Fig. 2, the input unit 12 and the output unit 16 may include hardware and/or software to enable the anomaly detection apparatus 10 to receive data from the X-ray detector 20, and to communicate with other devices and/or a network. For example, the input unit 12 may receive the unexposed image and associated metadata from the X-ray detector 20 via a wired connection or via a wireless connection. The output unit 16 may be embodied as a communications module for providing cellular telephone communications, and/or other data communications for the anomaly detection apparatus 10.

The processing unit(s) 12 may execute instructions to perform the method described herein, which will be explained in detail with respect to the embodiment shown in Fig. 4.

In some examples, the anomaly detection apparatus 10 may include a memory (not shown). The memory may include, but is not limited to, volatile memory and/or non-volatile memory. The memory may be used to store processor instructions, and other data and instructions to enable the processor to perform the techniques described herein.

Fig. 3 illustrates an alternate embodiment of an anomaly detection apparatus 10, where the anomaly detection apparatus 10 is included within the X-ray detector 20. The anomaly detection apparatus 10 may be substantially functionally equivalent to the apparatus 10 illustrated in Fig. 2.

Fig. 4 illustrates a flow chart describing a computer-implemented method 200 for identifying disturbance in an X-ray detector image caused by an external source, in accordance with an embodiment.

Beginning at block 210, i.e. step a), an apparatus, such as anomaly detection apparatus 10 shown in Fig. 2 or in Fig. 3, receives an unexposed image acquired by an X-ray detector and metadata associated with the acquired unexposed image.

The unexposed image may also be referred to as dark X-ray image. Dark X-ray images, often called offset frames, are acquired in regular time intervals by the X-ray detector to compensate the offset signal of each detector pixel in acquired radiated X-ray images. Dark images can be disturbed by artefacts (e.g. lines, clusters or waves) which are caused by different physical disturbances during the readout cycle of the X-ray detector. These can be electromagnetic or mechanical effects. The signature of the artefacts in the images depends on the signal form of the disturbance and the detector readout design (electronics, timing, etc.).

The image metadata may comprise information related to the medical institution, acquisition device, exam conditions, clinical protocol, and many other relevant pieces of information for acquiring the unexposed image. For example, the image metadata may include one or more of: date and time of acquiring the unexposed image, customer identifier, detector identifier, the examination type, position of the X-ray detector when acquiring the unexposed image, orientation of the X-ray detector when acquiring the unexposed image, motion of the X-ray detector when acquiring the unexposed image, and temperature of the X-ray detector when acquiring the unexposed image. The metadata may be obtained from the X-ray detector 20 and/or the X-ray imaging system.

At block 220, i.e., step b), the anomaly detection apparatus (e.g., the exemplary apparatus 10 shown in Fig. 2 or Fig. 3) analyses the received unexposed image to determine whether there is an anomaly in the received unexposed image that is indicative of an image disturbance caused by an external source, such as external electromagnetic or mechanical effects. The signature of the artefacts in the images depends on the signal form of the external disturbance and the detector readout design (electronics, timing, etc.). For example, the anomaly may include lines, clusters and/or waves artefacts in the image, which can interfere with relevant image information.

In some examples, the externally caused disturbance may be determined by comparing the received unexposed image with a set of reference unexposed images comprising one or more unexposed images that were previously acquired by the X-ray detector.

In some examples, it is also possible to analyse only the acquired unexposed image alone by an algorithm without a comparison. For example, stripe artefacts may be detected using a stripe detection algorithm.

It should be noted that lag, offset drift, and blinking pixels are detector typical artefacts, which do not belong to externally caused disturbance. Therefore, these artefacts are not indicative of an image disturbance caused by an external source.

At block 230, i.e., step c), in response to the detection of an anomaly that is indicative of an image disturbance caused by an external source, the anomaly detection apparatus (e.g., the exemplary apparatus 10 shown in Fig. 2 or Fig. 3) generates a single image anomaly report comprising the received unexposed image in full or data-size-reduced form and the received metadata. For data reduction, the image in full form may be cropped to the relevant area. It may be resampled to a lower image resolution. It may just consist of a few statistical values for each image area.

In some examples, the anomaly detection apparatus (e.g., the exemplary apparatus 10 shown in Fig. 2 or Fig. 3) may be further configured to analyse the detected anomaly and to provide analytical information about the detected anomaly to the single image anomaly report. In some examples, the analytical information about the detected anomaly comprises artefact type of the detected anomaly. Examples of the artefact type of the detected anomaly may include, but are not limited to, e.g. lines, clusters, and waves, which are caused by different physical disturbances during the readout cycle of the X-ray detector. These can be external electromagnetic or mechanical effects. The signature of the artefacts in the images depends on the signal form of the disturbance and the detector readout design (electronics, timing, etc.). In some examples, the analytical information may comprise size and location information about the detected anomaly. The location may be a useful information to find the anomaly and the location of the external source. In some examples, the analytical information may comprise a strength of the detected anomaly, which may be a useful information to determine the strength of the influence of the external source. In some examples, the analytical information may comprise signal frequencies derived from the artefact data.

In some examples, the anomaly detection apparatus may be further configured to create a thumbnail image of the detected anomaly and to provide the created thumbnail image of the detected anomaly to the single image anomaly report.

In some examples, the anomaly detection apparatus may be further configured to provide, via the output unit, instructions to prevent future anomalies. In some examples, the anomaly detection apparatus may comprise a lookup table or a database storing a plurality of abnormalities, each being associated with one or more actions to prevent future anomalies. These actions may be defined by one or more service technicians according to their experiences, or may be determined based on historic actions that were previously performed by one or more service technicians. The anomaly detection apparatus can then use the single image anomaly report to find the matching actions. In some examples, the anomaly detection apparatus may send the single image anomaly report to a server that stores a lookup table or database, and the server returns instructions to the anomaly detection apparatus.

The instructions may include one or more of the following action proposals:
- suggest to investigate an artefact that is not corrected well, so that correction of affected images can be improved;
- inform customer that a recently added device disturbs the detector;
- inform customer that the artefact he is experiencing is the same as other customers get from a specific device; and
- inform customer that he has disturbances on a regular schedule, e.g. each day at a specific time.

At block 240, i.e., step d), the output unit 16 is configured to provide the generated single image anomaly report, e.g., to a local, a network storage, and/or a cloud storage.

In other words, if an anomaly indicative of an externally caused disturbance is detected, a single image anomaly report is generated which may contain image sample, metadata (e.g., customer identifier, detector identifier, current time and date). The image sample may be a full offset-corrected dark image or some data-reduced version of it. The single image anomaly report may further comprise analytical information (e.g., artefact type, strength, size, and location of the anomaly) and/or instructions to prevent future anomalies. =

The anomaly detection apparatus and method as descried herein may provide one or more of the following benefits:
Artefacts caused by external sources, such as electromagnetic interference or mechanical shock/vibration, i.e., microphony, on the current generation of X-ray detectors, e.g., portable X-ray detectors, may remain in the clinical image and cannot be corrected by an offset frame. The anomaly detection apparatus and method as described herein can provide a comprehensive collection of artefact samples. These artefact samples may be usable for the development of a software for artefact detection and suppression.

It helps finding the source of sporadic artefacts in the clinical images, likely from electromagnetic sources, that only occur rarely. For example, one in several hundred images may be affected by sporadic artefacts, or less than one image per week. If the anomaly detection apparatus periodically acquires and checks unexposed images for artefacts and log detected artefacts, it is possible to have more data to identify the electromagnetic source, allowing to prevent further disturbances. The logs comprise metadata, which may contain additional available data like detector orientation, room and position in the room, enabling retrospective correlation to location or time of day, etc.

Further, no data privacy rules must be considered because the dark images do not contain any patient related information.

The generated single image anomaly reports may be useful for proactive corrective actions, and development of suppression algorithms. The generated single image anomaly reports may also be useable for customer complaint analysis.

Fig. 5 illustrates a block diagram of an exemplary system 100. The exemplary system 100 comprises an anomaly detection apparatus 10, a data storage 30, and an anomaly analysis apparatus 40.

The anomaly detection apparatus 10 may be substantially functionally equivalent to the apparatus illustrated in Fig. 2 or 3 to perform the method described herein, which is explained in detail with respect to the embodiment shown in Fig. 4. The anomaly detection apparatus 10 is configured to generate, for each unexposed image acquired by an X-ray detector 20 having an anomaly that is indicative of an image disturbance caused by an external source, a respective single image anomaly report and to provide the said single image anomaly report to the data storage 30. The metadata may comprise data obtained from the X-ray detector and the X-ray imaging system (e.g., examination type).

The data storage 30 may be any suitable data storage including, but not limited to, a local storage, a network storage, and a cloud storage. The data storage 30 is configured to store single image anomaly reports generated by the anomaly detection apparatus 10. It will be appreciated that the data storage 30 may collect single image anomaly reports from one or more X-ray detectors, although Fig. 5 shows a single X-ray detector by way of an example.

The anomaly analysis apparatus 40 may be embodied as, or in, a device or apparatus, such as a server, workstation, or mobile device. The anomaly analysis apparatus 40 may comprise a data analysis application, which may be a software application that enables a user to manipulate data obtained from the data storage 30. For example, the data analysis application may be a desktop application, a mobile application, or a web-based application. The data analysis application may comprise a user interface, such as an interactive interface including, but not limited to, a GUI, a character user interface and a touch screen interface. Via the software application, the user may access the data storage 30 to obtain the stored single image anomaly reports.

The anomaly analysis apparatus 40 is configured to analyse the single image anomaly reports stored in the data storage 30 and to generate an anomaly summary report comprising analytical information about detected anomalies stored in the data storage 30. In some examples, the anomaly summary report may comprise a list of detected anomalies stored in the data storage. In some examples, the anomaly summary report may comprise statistics comprising occurrence rate of anomalies, e.g., in total, or filtered e.g., per exam type, location, artefact type, and/or strength.

The anomaly detection apparatus 10, the data storage 30, and the anomaly analysis apparatus 40 may be associated with a network (not shown). In some examples, the network may be the internet. Alternatively, the network may be any other type and number of networks. For example, the network may be implemented by several local area networks connected to a wide area network. Of course, any other configuration and topology may be utilized to implement the network, including any combination of wired network, wireless networks, wide area networks, local area networks, etc.

Fig. 6 illustrates a flow chart describing a further example of the computer-implemented method 300. The exemplary computer-implemented method 300 shown in Fig. 6 further comprises the method steps performed by the data analysis apparatus 40.

Beginning at block 210, an anomaly detection apparatus 10 receives an unexposed image acquired by an X-ray detector and metadata associated with the acquired unexposed image in a similar way as block 210 illustrated in Fig. 4.

At block 220, the anomaly detection apparatus 10 analyses the received unexposed image to determine whether there is an anomaly in the received unexposed image that is indicative of an image disturbance caused by an external source in a similar way as block 220 illustrated in Fig. 4. In this example, the externally caused disturbance is determined by comparing the received unexposed image with a set of reference unexposed images comprising one or more unexposed images that were previously acquired by the X-ray detector. In some examples (not shown), it is also possible to analyse only the acquired unexposed image alone by an algorithm without a comparison. For example, stripe artefacts may be detected using a stripe detection algorithm.

In response to the detection of an anomaly that is indicative of an image disturbance caused by an external source, the anomaly detection apparatus 10 generates a single image anomaly report comprising the received unexposed image in full or data-size-reduced form and the received metadata at block 230.

Optionally, as shown in block 230a, for data reduction, the image in full form may be cropped to the relevant area. It may be resampled to a lower image resolution. It may just consist of a few statistical values for each image area. The singe image anomaly report may further comprise analytical information and/or thumbnail image of the artefact. Examples of the analytical information may include, but are not limited to, artefact type of the detected anomaly, size and location information about the detected anomaly, and a strength of the detected anomaly.

At block 240, the anomaly detection apparatus 10 provides the generated single image anomaly report, e.g., to a local or remote storage.

Optionally, as shown in block 240a, the anomaly detection apparatus 10 may further provide, via the output unit, feedback or instructions to prevent future anomalies. In some examples, the anomaly detection apparatus may store a lookup table or database storing a plurality of abnormalities, each being associated with one or more actions to prevent future anomalies. These actions may be defined by one or more service technicians according to their experiences, or may be determined based on historic actions that were previously performed by one or more service technicians. The anomaly detection apparatus can then use the single image anomaly report to find the matching actions. In some examples, the anomaly detection apparatus may send the single image anomaly report to a server that stores a lookup table or database, and the server returns instructions to the anomaly detection apparatus.

At block 250, the anomaly analysis apparatus 40 (e.g., a server, workstation, or mobile device) may extract the stored single image anomaly reports from the data storage, and generate an anomaly summary report comprising analytical information about detected anomalies stored in the data storage. In some examples, the anomaly summary report may comprise a list of detected anomalies stored in the data storage. In some examples, the anomaly summary report may comprise statistics comprising occurrence rate of anomalies, e.g., in total, or filtered e.g., per exam type, location, artefact type, and/or strength.

As shown in Fig. 6, the anomaly analysis apparatus 40 may extract the stored single image anomaly reports and generate the anomaly summary report on demand. For example, the anomaly analysis apparatus 40 may comprise a data analysis application, which may be a software application that enables a user, e.g., a field engineer, to manipulate data obtained from the data storage 30. For example, the data analysis application may be a desktop application, a mobile application, or a web-based application. The data analysis application may comprise a user interface, such as an interactive interface including, but not limited to, a GUI, a character user interface and a touch screen interface. Via the software application, the user may access the data storage to obtain the stored single image anomaly reports and the software application can then process the data accordingly.

It should also be understood that, unless clearly indicated to the contrary, in any methods claimed herein that include more than one step or act, the order of the steps or acts of the method is not necessarily limited to the order in which the steps or acts of the method are recited.

All definitions, as defined and used herein, should be understood to control over dictionary definitions, definitions in documents incorporated by reference, and/or ordinary meanings of the defined terms.

The indefinite articles "a" and "an," as used herein in the specification and in the claims, unless clearly indicated to the contrary, should be understood to mean "at least one."

The phrase "and/or," as used herein in the specification and in the claims, should be understood to mean "either or both" of the elements so conjoined, i.e., elements that are conjunctively present in some cases and disjunctively present in other cases. Multiple elements listed with "and/or" should be construed in the same fashion, i.e., "one or more" of the elements so conjoined. Other elements may optionally be present other than the elements specifically identified by the "and/or" clause, whether related or unrelated to those elements specifically identified.

As used herein in the specification and in the claims, "or" should be understood to have the same meaning as "and/or" as defined above. For example, when separating items in a list, "or" or "and/or" shall be interpreted as being inclusive, i.e., the inclusion of at least one, but also including more than one, of a number or list of elements, and, optionally, additional unlisted items. Only terms clearly indicated to the contrary, such as "only one of' or "exactly one of," or, when used in the claims, "consisting of," will refer to the inclusion of exactly one element of a number or list of elements. In general, the term "or" as used herein shall only be interpreted as indicating exclusive alternatives (i.e. "one or the other but not both") when preceded by terms of exclusivity, such as "either," "one of," "only one of," or "exactly one of."

As used herein in the specification and in the claims, the phrase "at least one," in reference to a list of one or more elements, should be understood to mean at least one element selected from any one or more of the elements in the list of elements, but not necessarily including at least one of each and every element specifically listed within the list of elements and not excluding any combinations of elements in the list of elements. This definition also allows that elements may optionally be present other than the elements specifically identified within the list of elements to which the phrase "at least one" refers, whether related or unrelated to those elements specifically identified.

In the claims, as well as in the specification above, all transitional phrases such as "comprising," "including," "carrying," "having," "containing," "involving," "holding," "composed of," and the like are to be understood to be open-ended, i.e., to mean including but not limited to. Only the transitional phrases "consisting of' and "consisting essentially of' shall be closed or semi-closed transitional phrases, respectively.

In another exemplary embodiment of the present invention, a computer program or a computer program element is provided that is characterized by being adapted to execute the method steps of the method according to one of the preceding embodiments, on an appropriate system.

The computer program element might therefore be stored on a computer unit, which might also be part of an embodiment of the present invention. This computing unit may be adapted to perform or induce a performing of the steps of the method described above. Moreover, it may be adapted to operate the components of the above described apparatus. The computing unit can be adapted to operate automatically and/or to execute the orders of a user. A computer program may be loaded into a working memory of a data processor. The data processor may thus be equipped to carry out the method of the invention.

This exemplary embodiment of the invention covers both, a computer program that right from the beginning uses the invention and a computer program that by means of an up-date turns an existing program into a program that uses the invention.

Further on, the computer program element might be able to provide all necessary steps to fulfil the procedure of an exemplary embodiment of the method as described above.

According to a further exemplary embodiment of the present invention, a computer readable medium, such as a CD-ROM, is presented wherein the computer readable medium has a computer program element stored on it which computer program element is described by the preceding section.

A computer program may be stored and/or distributed on a suitable medium, such as an optical storage medium or a solid state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

## Claims

1. An anomaly detection apparatus (10) for identifying disturbance in an X-ray detector image caused by an external source, comprising:
- an input unit (12);
- a processing unit (14); and
- an output unit (16);
wherein the input unit is configured to receive an unexposed image acquired by an X-ray detector and metadata associated with the acquired unexposed image;
wherein the processing unit is configured to analyse the received unexposed image to determine whether there is an anomaly in the received unexposed image that is indicative of an image disturbance caused by an external source, and in response to the detection of an anomaly that is indicative of an image disturbance caused by an external source, to generate a single image anomaly report comprising the received unexposed image in full or data-size-reduced form and the received metadata; and
wherein the output unit is configured to provide the generated single image anomaly report.

2. The anomaly detection apparatus according to claim 1,
wherein the processing unit is configured to determine the anomaly in the received unexposed image by comparing the received unexposed image with a set of reference unexposed images comprising one or more unexposed images that were previously acquired by the X-ray detector.

3. The anomaly detection apparatus according to claim 1 or 2,
wherein the processing unit is further configured to analyse the detected anomaly and to provide analytical information about the detected anomaly to the single image anomaly report.

4. The anomaly detection apparatus according to claim 3,
wherein the analytical information about the detected anomaly comprises one or more of:
- artefact type of the detected anomaly;
- signal frequencies derived from the artefact data;
- size and location information about the detected anomaly; and
- strength of the detected anomaly.

5. The anomaly detection apparatus according to any one of the preceding claims,
wherein the processing unit is further configured to create a thumbnail image of the detected anomaly and to provide the created thumbnail image of the detected anomaly to the single image anomaly report.

6. The anomaly detection apparatus according to any one of the preceding claims,
wherein the processing unit is further configured to provide, via the output unit, an instruction to prevent future anomalies.

7. The anomaly detection apparatus according to claim 6,
wherein the instruction comprises one or more of the following action proposals:
- suggest to investigate an artefact that is not corrected well, so that correction of affected images can be improved;
- inform customer that a recently added device disturbs the detector;
- inform customer that the artefact he is experiencing is the same as other customers get from a specific device; and
- inform customer that he has disturbances on a regular schedule.

8. The anomaly detection apparatus according to any one of the preceding claims,
wherein the processing unit is configured to provide the single image anomaly report to a data storage.

9. The anomaly detection apparatus according to any one of the preceding claims,
wherein the metadata comprises one or more of:
- date and time of acquiring the unexposed image;
- customer identifier;
- detector identifier;
- the examination type;
- position of the X-ray detector when acquiring the unexposed image;
- orientation of the X-ray detector when acquiring the unexposed image;
- motion of the X-ray detector when acquiring the unexposed image; and
- temperature of the X-ray detector when acquiring the unexposed image.

10. An X-ray detector (20), comprising:
- an anomaly detection apparatus according to any one of the preceding claims.

11. A system (100), comprising:
- an anomaly detection apparatus (10) for identifying disturbance in an X-ray detector image caused by an external source according to any one of claims 1 to 9;
- a data storage (30); and
- an anomaly analysis apparatus (40);
wherein the anomaly detection apparatus is configured to generate, for each unexposed image acquired by an X-ray detector having an anomaly that is indicative of an image disturbance caused by an external source, a respective single image anomaly report and to provide the said single image anomaly report to the data storage; and
wherein the anomaly analysis apparatus is configured to analyse the single image anomaly reports stored in the data storage and to generate an anomaly summary report comprising analytical information about detected anomalies stored in the data storage.

12. The system according to claim 11,
wherein the anomaly summary report comprises one or more of:
- list of detected anomalies stored in the data storage; and
- statistics comprising occurrence rate of anomalies.

13. An anomaly detection method (200) for identifying disturbance in an X-ray detector image caused by an external source, comprising:
a) receiving (210) an unexposed image acquired by an X-ray detector and metadata associated with the acquired unexposed image;
b) analysing (220) the received unexposed image to determine whether there is an anomaly in the acquired unexposed image that is indicative of an image disturbance caused by an external source;
c) in response to the detection of an anomaly that is indicative of an image disturbance caused by an external source, generating (230) a single image anomaly report comprising the received unexposed image in full or data-size-reduced form and the received metadata; and
d) providing (240) the generated single image anomaly report.

14. A computer program comprising instructions which, when the program is executed by a computer, cause the computer to carry out steps according to method of claim 13.

15. A computer-readable medium having stored thereon the computer program of claim 14.
